# EUROPEAN PATENT SPECIFICATION

(11) **EP 3 327 546 B1**
(45) Date of publication and mention of the grant of the patent: **06.11.2019**
(21) Application number: 17171212.8
(22) Date of filing: 16.05.2017
(51) Int. Cl.: G06F 3/01, G01B 11/25, G02B 27/42, H04N 5/225

(54) **INTERACTIVE DISPLAY DEVICE AND INTERACTIVE DISPLAY SYSTEM**
VORRICHTUNG ZUR INTERAKTIVEN ANZEIGE UND SYSTEM ZUR INTERAKTIVEN ANZEIGE
DISPOSITIF ET SYSTÈME D'AFFICHAGE D'IMAGES INTERACTIVES

(30) Priority: 24.11.2016 TW 105138621
(43) Date of publication of application: 30.05.2018
(73) Proprietor: Industrial Technology Research Institute, Chutung, Hsinchu 31040 (TW)
(72) Inventor: LUO, Shih-Bin, 31040 Hsinchu (TW); LIN, Chun-Chuan, 31040 Hsinchu (TW)
(74) Representative: Manitz Finsterwald Patent- und Rechtsanwaltspartnerschaft mbB

(56) References cited:
- US-A1- 2009 096 783
- US-A1- 2012 249 416
- US-A1- 2015 302 648
- US-A1- 2016 140 766

## Description

### CROSS-REFERENCE TO RELATED APPLICATION

The present disclosure is based on, and claims priority from Taiwan Application Number 105138621, filed on November 24, 2016.

### BACKGROUND

### 1. Technical Field

The present disclosure relates to display devices and display systems, and, especially, to an interactive display device and an interactive display system having image positioning capability.

### 2. Description of the related art

The emergence and popularization of head-mounted display (HMD) or smart glasses (such as Google glass) has been widely used in various fields, such as maintenance, disaster relief, medical treatment, surgery and other special fields. In these applications, the user can use the head-mounted display or smart glasses to obtain the immediate image, and transmit the image to remote ends to communicate with them. However, the user and the remote ends cannot accurately communicate with the image alone. For example, the precise location to be repaired or to be rescued cannot be described, but only a rough location indicated by up, down, left, and right directions with oral expressions. This not only results in longer communication time between the two sides, but also is more likely to cause time delay for repair or rescue.

US 2009/0096783 A1 discloses an apparatus for 3D mapping of an object including an illumination assembly having a coherent light source and a diffuser, which are arranged to project a primary speckle pattern on the object. A single image capture assembly is arranged to capture images of the primary speckle pattern on the object from a single, fixed location and angle relative to the illumination assembly. A processor is coupled to process the images of the primary speckle pattern captured at the single, fixed angle so as to derive a 3D map of the object.

US 2012/0249416 A1 discloses various embodiments including systems and methods for rendering images in a virtual or augmented reality system that may include capturing scene images of a scene in a vicinity of a first and a second projector, capturing spatial data with a sensor array in the vicinity of the first and second projectors, analyzing captured scene images to recognize body parts, and projecting images from each of the first and second projectors with a shape and orientation determined based on the recognized body parts. Additional rendering operations may include tracking movements of the recognized body parts, applying a detection algorithm to the tracked movements to detect a predetermined gesture, applying a command corresponding to the detected predetermined gesture, and updating the projected images in response to the applied command.

US 2015/0302648 A1 discloses a multi dynamic environment and location based active augmented reality (AR) system. Scanning and mapping are performed from the perspective of a user wearing a head mounted display (HMD) in the physical environment. Systems and methods are described herein for projecting structured light onto a physical environment and capturing reflections of the structured light to map the environment.

US 2016/0140766 A1 discloses a surface projection system for augmented reality. The surface projection system includes a surface projection device that is positionable adjacent a surface and having a light element and a sensor. The light element is configured to project a reference pattern on the surface. The sensor is positioned adjacent the surface and configured to gaze along the surface.

Therefore, how to provide an interactive display device and an interactive display system having image positioning capability is one of the urgent issues to be solved.

### SUMMARY

The invention is set out in the appended set of claims.

### BRIEF DESCRIPTION OF THE DRAWINGS

FIG. 1 is a three-dimensional schematic diagram illustrating a first example not covered by the appended set of claims of an interactive display device according to the present disclosure;
FIG. 2 is a three-dimensional schematic diagram illustrating a second example not covered by the appended set of claims of an interactive display device according to the present disclosure;
FIGs. 3A and 3B are schematic diagrams of an interactive display device according to the present disclosure;
FIG. 4 is a three-dimensional schematic diagram illustrating an embodiment of an interactive display device according to the present disclosure;
FIG. 5 is a functional block diagram of an interactive display system according to the present disclosure;
FIG. 6 is a schematic diagram of an interactive display system according to the present disclosure;
FIG. 7 is a schematic diagram of an interactive display system of a fourth example not covered by the appended set of claims according to the present disclosure;
FIG. 8 is a schematic diagram of an interactive display system of a fifth example not covered by the appended set of claims according to the present disclosure;
FIG. 9 is a schematic diagram of an interactive display system of a sixth example not covered by the appended set of claims according to the present disclosure; and
FIG. 10 is a schematic diagram of an interactive display system of a seventh example not covered by the appended set of claims according to the present disclosure.

### DETAILED DESCRIPTION

One skilled in the art can readily comprehend other advantages and efficacies of the present disclosure in view of the disclosure of this specification. The present disclosure can also be implemented or applied by other specific examples.

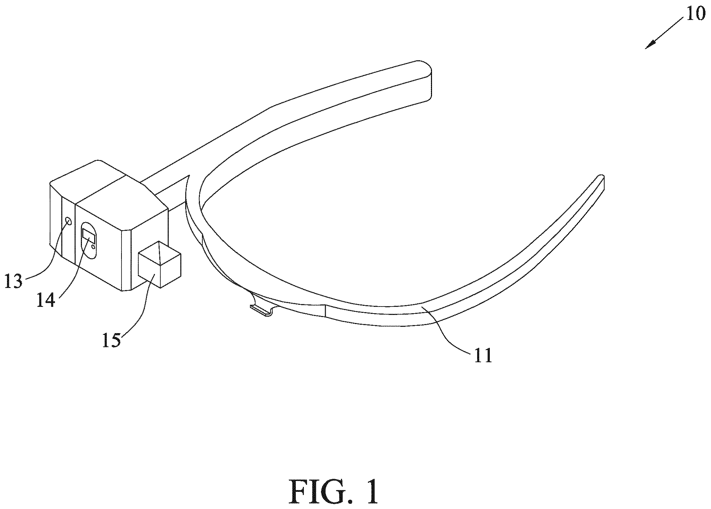

Referring to FIG. 1, an interactive display device 10 of a first example not covered by the appended set of claims according to the present disclosure includes a body 11, an optical project unit 13, an image capture unit 14, and a display unit 15. The body 11 may be an eyeglass, a mobile phone or a tablet computer. In an example not covered by the appended set of claims, the body 11 will be described as an eyeglass, but the present disclosure is not limited thereto.

As shown in FIG. 1, the optical project unit 13 is provided on the body 11, for example, on the right stand of a spectacle frame. The body 11 is provided with a microprocessor (not shown), and the optical project unit 13 is controlled by a microprocessor and can project a pattern to a scene.

The image capture unit 14 is also provided on the body 11, for example, on the right stand of a spectacle frame. The image capture unit 14 is also controlled by the microprocessor. After the optical project unit 13 projects the pattern to the scene, the image capture unit 14 captures the image containing the pattern in the scene.

The display unit 15 is also provided on the body 11, for example, in front of the position of the right lens of a spectacle. When a user wears the interactive display device 10, the right eye of the user can see the image displayed by the display unit 15. The arrangement positions of the optical project unit 13, the image capture unit 14, and the display unit 15 are merely examples, and the present disclosure is not limited thereto.

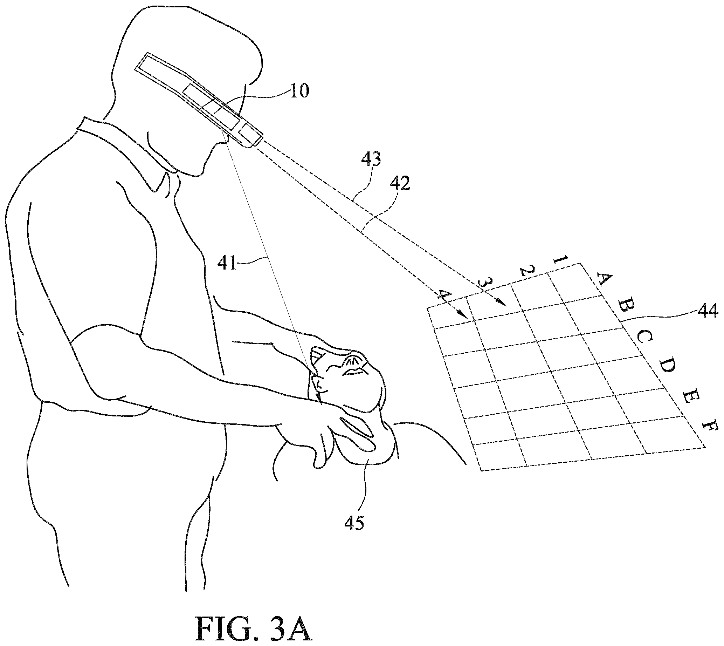

In an example not covered by the appended set of claims, the optical project unit 13 is a laser having a laser light source and a diffraction element, wherein the diffraction element may be an optical hologram or a microstructural optical film. The optical project unit 13 may project a pattern through a laser light source and a diffraction element, and the pattern is a checkerboard pattern or a concentric circle pattern. As shown in FIG. 3A, the pattern 44 is a checkerboard pattern, where the horizontal axis is marked by A to F and the vertical axis is marked by 1 to 4. Nevertheless, the present disclosure is not limited to the form of the pattern, nor the designations of the vertical and horizontal axes.

In an example not covered by the appended set of claims, the image capture unit 14 is a camera, which may comprise a color complementary metal-oxide-semiconductor (CMOS) image sensor or a color-coupled device (CCD) image sensor, but the disclosure is not limited thereto.

In an example not covered by the appended set of claims, the display unit 15 is a micro-display panel, such as a liquid-crystal display (LCD) or a panel produced by liquid crystal on silicon (LCOS) technology, with a virtual image magnifying optical element in the panel, which can magnify an image captured by the image capture unit 14 to a degree that users can view.

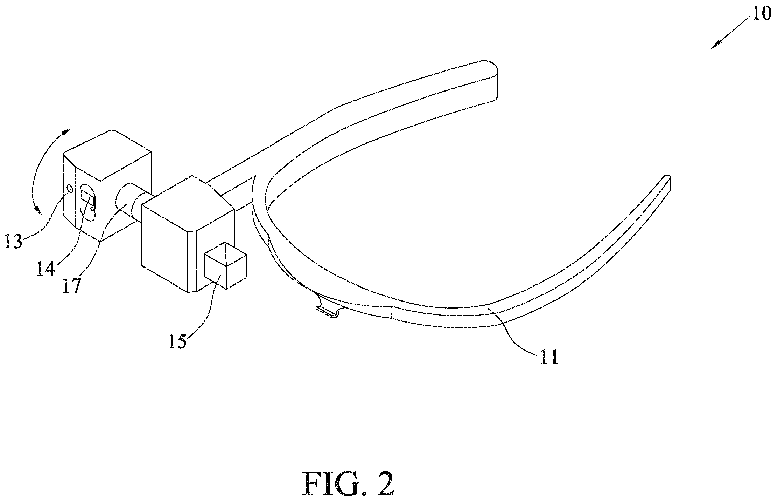

Please refer to the interactive display device 10 of a second example not covered by the appended set of claims according to the present disclosure illustrated by FIG. 2. Only the difference between the second example not covered by the appended set of claims and the first example not covered by the appended set of claims will be described. The interactive display device 10 of the second example not covered by the appended set of claims according to the present disclosure is different from the first example not covered by the appended set of claims in that the optical project unit 13 and the image capture unit 14 are not directly provided on the body 11. In an example not covered by the appended set of claims, the interactive display device 10 further includes an angle adjustment unit 17 provided on the body 11, for example, on a right stand of a spectacle frame, and the optical project unit 13 and the image capture unit 14 are connected to the body 11 through the angle adjustment unit 17.

In an example not covered by the appended set of claims, the angle adjustment unit 17 is a spindle motor, such that the optical projection unit 13 and the image pickup unit 14 can simultaneously rotate with respect to the body 11. The rotation method may be that the microprocessor inside the body 11 controls the spindle motor according to a tilt angle detected by a gyroscope inside the body 11 to further control the rotation angle of the optical project unit 13 and the image capture unit 14.

In another example not covered by the appended set of claims, the angle adjustment unit 17 may be a rotary shaft without power, and a user may manually adjust the rotation angles of the optical project unit 13 and the image capture unit 14.

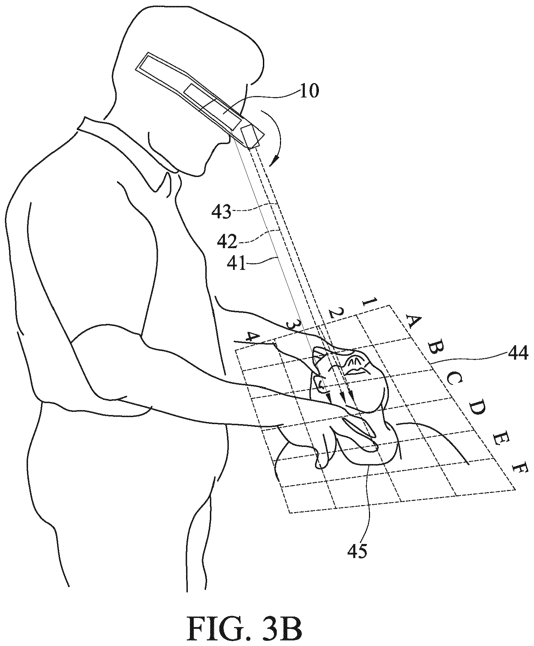

The purpose of providing the angle adjustment unit 17 in the interactive display device 10 of the present disclosure is to align the line of sight of a user with the pattern projection direction of the optical project unit and the image capture direction of the image capture unit. Referring to FIG. 3A, after a user wears the interactive display device 10 according to the present disclosure and plans to project a pattern 44 in a scene 45, if the interactive display device 10 is not provided with the angle adjustment unit 17, the sight line 41 of the user is liable to be inconsistent with the pattern projection direction 42 of the optical project unit 13 and the image capture direction 43 of the image capture unit 14 when the user bows the head to look at the near scene 45. This is due to the viewing angle of the human eye is considerably large. To accurately project the pattern 44 onto the scene 45, the user obviously has to take a lower head posture, which is likely to cause neck discomfort. Therefore, as shown in FIG. 3B, the interactive display device 10 according to the present disclosure is provided with an angle adjustment unit 17 that projects the pattern 44 to the scene 45 by rotating the optical project unit 13 and the image capture unit 14. The user does not have to take a lower head position, and hence the interactive display device 10 does not incur a burden on the user.

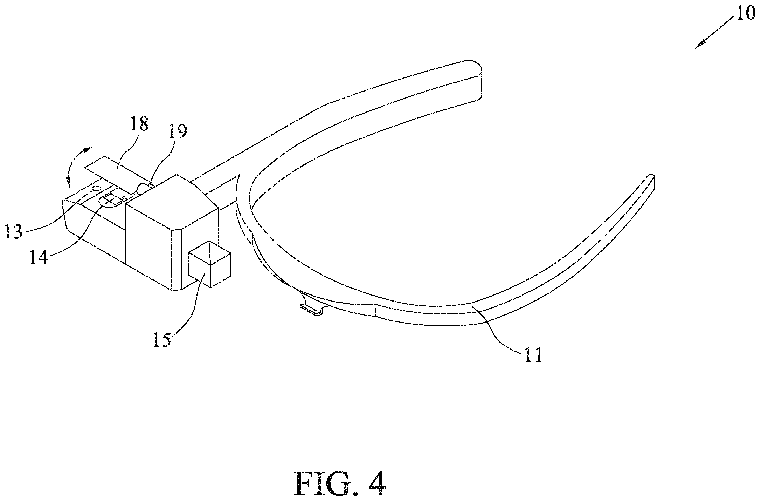

Please refer to the interactive display device 10 of an embodiment according to the present disclosure illustrated in FIG. 4. Only the difference between this embodiment and the first example will be described. The interactive display device 10 of this embodiment according to the present disclosure is different from the first example in that the interactive display device 10 of this embodiment further comprises a reflection unit 18. The reflection unit 18 is provided on the body 11 through a spindle motor 19, for example, on a right stand of a spectacle frame. In an embodiment, the optical project unit 13 and the image capture unit 14 are provided on the right stand of the spectacle frame, but below the reflection unit 18. That is, the reflection unit 18 is provided in the proceeding path of the pattern projection direction of the optical project unit 13 and the image capture direction of the image capture unit 14. In an embodiment, the reflective unit 18 may be a reflecting mirror, and may be used to change the pattern projection direction and the image capture direction. The method is that a microprocessor within the body 11 controls the spindle motor 19 according to a tilt angle detected by a gyroscope provided within the body 11 to further control the rotation angle of the reflection unit 18.

In an embodiment, the interactive display device 10 may further comprise a wireless transmission unit (not shown) for transmitting images to a terminal unit to display. In an embodiment, the wireless transmission unit may be configured to use Bluetooth, Wi-Fi, 3G or 4G wireless network transmission, but the disclosure is not limited thereto.

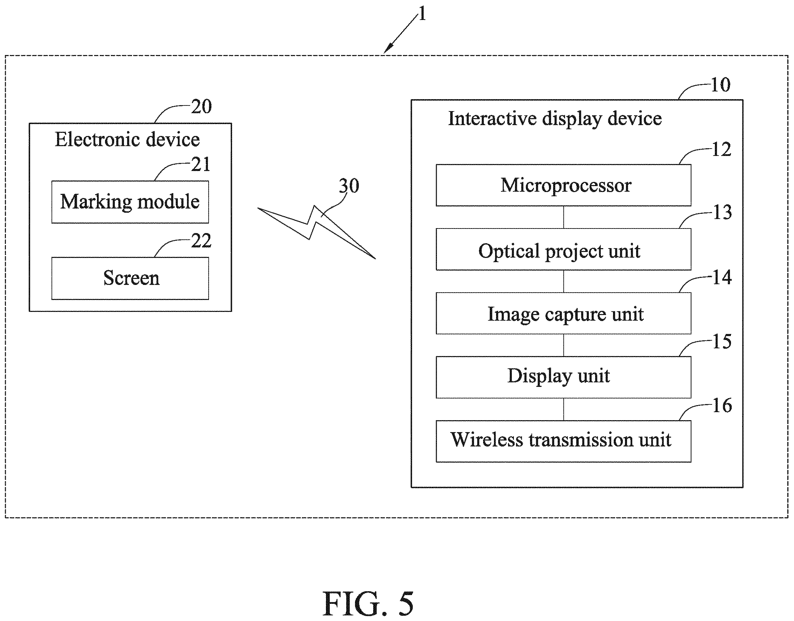

Referring to FIGs. 1 and 5, the interactive display system 1 according to the present disclosure comprises an interactive display device 10 and an electronic device 20. The interactive display device 10 comprises a body 11, an optical project unit 13, an image capture unit 14, and a display unit 15. The body 11 of the interactive display device 10 is provided with a microprocessor 12 for controlling the optical project unit 13 and the image capture unit 14. The detailed technical content of the interactive display device 10 has been described above and will not be described in detail here.

The electronic device 20 is connected to a wireless transmission unit 16 of the interactive display device 10 via a network 30 for receiving an image containing a pattern captured by the image capture unit 14 of the interactive display device 10. The electronic device 20 comprises a screen 22 on which images containing the patterns captured by the image capture unit 14 of the interactive display device 10 are displayed.

In an embodiment, the electronic device 20 is a mobile phone, tablet or desktop computer with a processor, and the network may be a Bluetooth, Wi-Fi, 3G or 4G wireless network.

In an embodiment, the electronic device 20 comprises a marking module 21. The module according to the present disclosure is a software that can be executed by a processor. The marking module 21 is used to mark an image, for example, editing the image by using an image software such as MS Paint. The tagged image can be transmitted via the network 30 to the interactive display device 10 and displayed on the display unit 15.

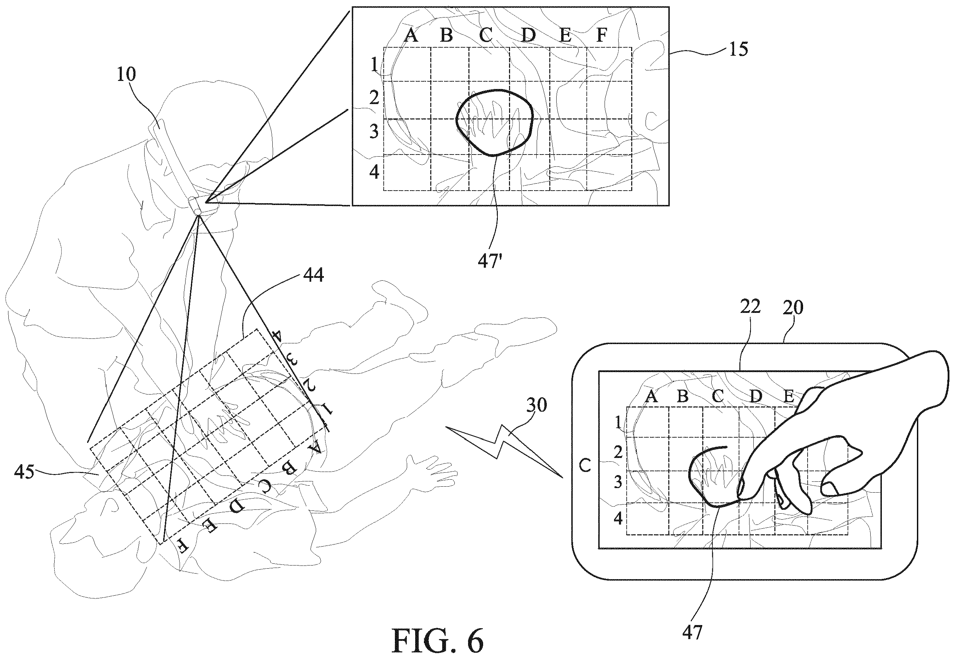

As shown in FIG. 6, when the interactive display system 1 according to the present disclosure is applied, a user wears the interactive display device 10 and projects a pattern 44 on a scene 45 (for example, a casualty), and the user can see the image containing the pattern 44 in the display unit 15. The interactive display device 10 transfers the captured image containing the pattern 44 to the electronic device 20 through the network 30 and displays the image on the screen 22 of the electronic device 20. The user viewing the electronic device 20 can easily communicate with the user wearing the interactive display device 10 based on the pattern 44 because the image contains the pattern 44 (for example, a checkerboard pattern), and both parties have common coordinate cognition. For example, the position of pressing C3 may be indicated. In an embodiment, the user viewing the electronic device 20 may make a mark 47 to the image on the screen 22 (e.g., draw a circle at the C3 position), and the marked image may be transmitted to the interactive display device 10 through the network 30 and displayed on the display unit 15. The user wearing the interactive display device 10 can see the mark 47' in the image on the display unit 15. The precise position can be determined by the marks 47 and 47' in the images such that communications of both sides can go more smoothly.

Although the interactive display device 10 according to the present disclosure is explained by taking an example of the body 11 as a spectacle, the body 11 of the interactive display device 10 according to the present disclosure may be a mobile phone or a tablet computer, the optical project unit 13 may be a plug-in project unit, the image capturing unit 14 may be a camera on a mobile phone or a tablet computer, and the display unit 15 may be a screen on a mobile phone or a tablet computer. The present disclosure is not limited thereto.

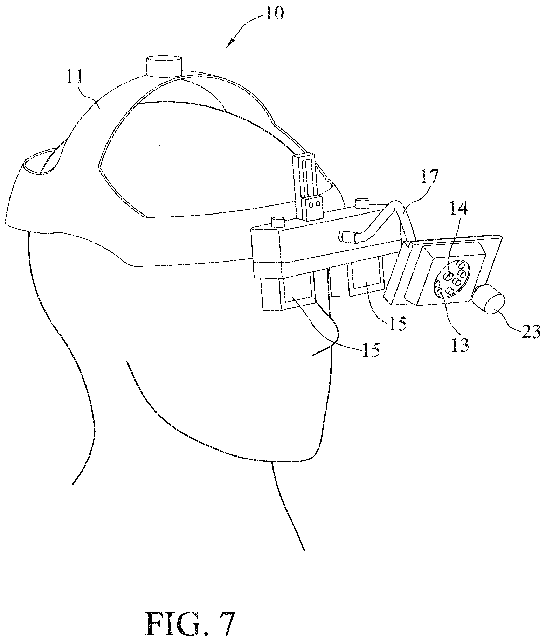

Please refer to FIG. 7. An interactive display device 10 of a fourth example not covered by the appended set of claims according to the present disclosure comprises a body 11, an optical project unit 13, an image capture unit 14, and a display 15. In an example not covered by the appended set of claims, the body 11 is a helmet. In the following, the present disclosure is described as the body 11 is exemplified by, but not limited to, a helmet.

The display unit 15 is disposed on the body 11. In an example not covered by the appended set of claims, the interactive display device 10 may have two display units 15 disposed at positions in front of two eyes of a user, to allow the user to view the image displayed on the display units 15 with two eyes, respectively.

The optical project unit 13 and the image capture unit 14 may be connected to the body 11 via the angle adjustment unit 17. In an example not covered by the appended set of claims, the angle adjustment unit 17 may have a bendable structure such as a plastic soft pipe having a wire embedded therein. The angle adjustment unit 17 allows the optical project unit 13 and the image capture unit 14 to be fixed to a specific position with respect to the body 11 at the same time. Therefore, a user is allowed to use the angle adjustment unit 17 to adjust the pattern projection direction of the optical project unit 13 and the image capture direction of the image capture unit 14.

In an example not covered by the appended set of claims, the optical project unit 13 is, but not limited to, a light emitting diode, or a laser having a laser light source and a diffraction element. In another example not covered by the appended set of claims, the optical project unit 13 surrounds the image capture unit 14, and uses a knob 23 to adjust the intensity of light emitted by the optical project unit 13 such as a light emitting diode.

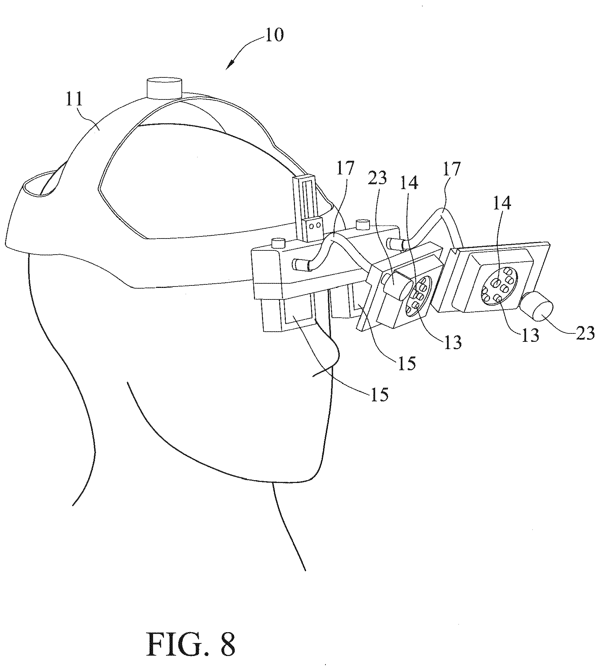

Please refer to FIG. 8, which illustrates an interactive display device 10 of a fifth example not covered by the appended set of claims according to the present disclosure. The fifth example not covered by the appended set of claims differs from the fourth example not covered by the appended set of claims in the number of the angle adjustment unit 17, the optical project unit 13 and the image capture unit 14. The interactive display device 10 shown in FIG. 8 has two angle adjustment units 17, two optical project units 13 and two image capture units 14, and can constitute 3D images, to allow a user to view 3D images via the display unit 15.

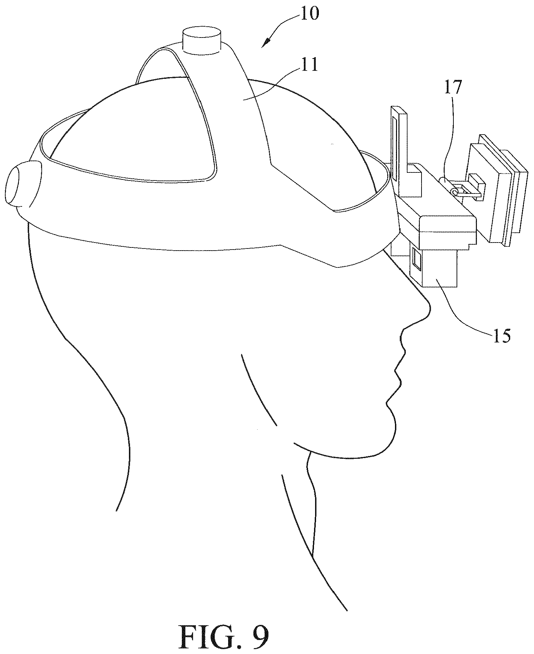

Please refer to FIG. 9, which illustrates an interactive display device 10 of a sixth example not covered by the appended set of claims according to the present disclosure. The sixth example not covered by the appended set of claims differs from the fourth example not covered by the appended set of claims of FIG. 7 in that the angle adjustment unit 17 of the sixth example not covered by the appended set of claims can be a clamshell-type structure pivotally connected to the body 11.

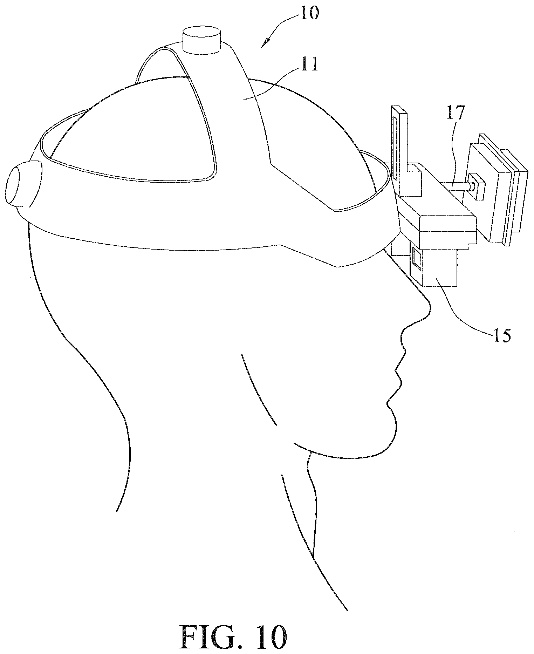

Please refer to FIG. 10, which illustrates an interactive display device 10 of a seventh example not covered by the appended set of claims according to the present disclosure. The seventh example not covered by the appended set of claims differs from the fourth example not covered by the appended set of claims of FIG. 7 in that the angle adjustment unit 17 of the seventh example not covered by the appended set of claims can be universal bearing.

The interactive display device 10 according to the present disclosure allows a user to view images by aiming the eyes directly on the display unit 15 when using the optical project unit 13 and the image capture unit 14 to capture images, without bowing the head to look downward. A user can easily and quickly manipulate the interactive display device 10 according to the present disclosure.

With the interactive display device and system according to the present disclosure, the optical project unit of the interactive display device can project a pattern, and the image capture unit of the interactive display device can capture an image containing the pattern, which can be transmitted to an electronic device, such that both sides of users can precisely position through the images containing the pattern. Moreover, users can also mark the images containing the pattern by a marking module of an electronic device. Therefore, the present disclosure has an effect of easy communication, and allows a user to manipulate with the eyes and hands at his will and ease. In the application, young doctors can wear the interactive display device of the present disclosure for examining patients, and senior doctors can assist in determining patients' conditions by images containing a pattern displayed by the electronic device or mark the images for facilitating the communication between the two sides. The present disclosure can further be applied in the fields of maintenance, disaster relief, medical treatment and surgery, including watch/clock maintenance, vehicle maintenance, oral treatment, intravenous injection, surgery, cooking teaching guidance, eyelash extension and nail painting, as a bridge between the first-line user and the back-end support center.

The embodiments described above are only illustrative of the technical principles, features, and effects of the present disclosure, and are not intended to limit the scope of the present disclosure. Any person skilled in the art can, without departing from the scope of the present disclosure, modify and change the embodiments. The scope of protection of the present disclosure is set forth in the following claims.

## Claims

1. An interactive display device (10), comprising:
a body (11) having a microprocessor (12) therein;
an optical project unit (13) provided on the body (11) and controlled by the microprocessor (12) for projecting a pattern (44) to a scene (45);
an image capture unit (14) provided on the body (11) and controlled by the microprocessor (12) for capturing an image containing the pattern (44) in the scene (45);
a display unit (15) provided on the body (11) configured for displaying the image;
**characterized in that** the interactive display device (10) further comprises: a reflecting unit (18) provided on the body (11) and located in a pattern projection direction (42) of the optical project unit (13) and an image capture direction (43) of the image capture unit (14) configured for changing the pattern projection direction (42) and the image capture direction (43).

2. The interactive display device (10) according to claim 1, wherein the body (11) is an eyeglass, a mobile phone or a tablet computer.

3. The interactive display device (10) according to claim 1, wherein the optical project unit (13) is a light emitting diode, or a laser having a laser light source and a diffraction element.

4. The interactive display device (10) according to claim 3, wherein the diffraction element is an optical whole-picture or a microstructural optical film.

5. The interactive display device (10) according to claim 1, wherein the pattern (44) is a checkerboard pattern or a concentric circle pattern.

6. The interactive display device (10) according to claim 1, wherein the image capture unit (14) is a camera including a color complementary metal semiconductor image sensor or a color photosensitive coupling element image sensor.

7. The interactive display device (10) according to claim 1, further comprising a wireless transmission unit (16) configured for transmitting the image to a terminal unit to display, wherein the wireless transmission unit (16) uses Bluetooth, Wi-Fi, 3G or 4G wireless network transmission.

8. The interactive display device (10) according to claim 1, wherein the reflecting unit (18) is provided on the body (11) through a spindle motor, and the microprocessor (12) is configured to control the spindle motor in accordance with a gyroscope provided in the body (11) to control a rotation angle of the reflection unit (18).

9. An interactive display system (1) comprising:
the interactive display device (10) according to claim 1 and further comprising:
a wireless transmission unit (16);
the system (1) further comprising an electronic device (20) connected to the wireless transmission unit (16) of the interactive display device (10) through a network (30) to receive and display the image on a screen (22) of the electronic device (20),

10. The interactive display system (1) according to claim 9, wherein the body (11) is an eyeglass, a mobile phone or a tablet computer, and the electronic device (20) is a mobile phone, a tablet computer or a desktop computer.

11. The interactive display system (1) according to claim 9, wherein the optical project unit (13) is a light emitting diode, or a laser having a laser light source and a diffraction element.

12. The interactive display system (1) according to claim 11, wherein the diffraction element is an optical whole-picture or a microstructural optical film.

13. The interactive display system (1) according to claim 9, wherein the pattern (44) is a checkerboard pattern or a concentric circle pattern.

14. The interactive display system (1) according to claim 9, wherein the image capture unit (14) is a camera including a color complementary metal semiconductor image sensor or a color photosensitive coupling element image sensor.

15. The interactive display system (1) according to claim 9, wherein the network (30) is a Bluetooth, a Wi-Fi, 3G or 4G wireless network.

16. The interactive display system (1) according to claim 9, wherein the reflecting unit (18) is provided on the body (11) through a spindle motor, and the microprocessor (12) is configured to control the spindle motor in accordance with a gyroscope provided in the body (11) to control a rotation angle of the reflection unit (18).

17. The interactive display system (1) according to claim 9, wherein the electronic device (20) comprises a marking module (21) configured for marking the image and the marked image can be transmitted to the interactive display device (10) through the network (30) and displayed on the display unit (15) of the interactive display device (10).

## Patentansprüche

1. Interaktive Anzeigevorrichtung (10), umfassend:
einen Körper (11) mit einem Mikroprozessor (12) darin;
eine optische Projektionseinheit (13), die an dem Körper (11) vorgesehen ist und durch den Mikroprozessor (12) gesteuert wird, um ein Muster (44) auf eine Szene (45) zu projizieren;
eine Bilderfassungseinheit (14), die an dem Körper (11) vorgesehen ist und durch den Mikroprozessor (12) gesteuert wird, um ein Bild zu erfassen, das das Muster (44) in der Szene (45) enthält;
eine an dem Körper (11) vorgesehene Anzeigeeinheit (15), die zum Anzeigen des Bildes konfiguriert ist;
**dadurch gekennzeichnet, dass** die interaktive Anzeigevorrichtung (10) ferner umfasst:
eine Reflexionseinheit (18), die an dem Körper (11) vorgesehen ist und in einer Musterprojektionsrichtung (42) der optischen Projektionseinheit (13) und einer Bilderfassungsrichtung (43) der Bilderfassungseinheit (14) angeordnet ist und zum Ändern der Musterprojektionsrichtung (42) und der Bilderfassungsrichtung (43) konfiguriert ist.

2. Interaktive Anzeigevorrichtung (10) nach Anspruch 1, wobei der Körper (11) eine Brille, ein Mobiltelefon oder ein Tablet-Computer ist.

3. Interaktive Anzeigevorrichtung (10) nach Anspruch 1, wobei die optische Projektionseinheit (13) eine lichtemittierende Diode oder ein Laser mit einer Laserlichtquelle und einem Beugungselement ist.

4. Interaktive Anzeigevorrichtung (10) nach Anspruch 3, wobei das Beugungselement ein optisches Gesamtbild oder ein mikrostruktureller optischer Film ist.

5. Interaktive Anzeigevorrichtung (10) nach Anspruch 1, wobei das Muster (44) ein Schachbrettmuster oder ein Muster mit konzentrischen Kreisen ist.

6. Interaktive Anzeigevorrichtung (10) nach Anspruch 1, wobei die Bilderfassungseinheit (14) eine Kamera ist, die einen farbkomplementären Metall-Halbleiter-Bildsensor oder einen farbphotoempfindlichen Koppelelement-Bildsensor beinhaltet.

7. Interaktive Anzeigevorrichtung (10) nach Anspruch 1, ferner umfassend eine drahtlose Übertragungseinheit (16), die zum Übertragen des Bildes an eine Endgeräteeinheit zur Anzeige konfiguriert ist, wobei die drahtlose Übertragungseinheit (16) Bluetooth, Wi-Fi, 3G oder 4G zur drahtlosen Netzwerkübertragung verwendet.

8. Interaktive Anzeigevorrichtung (10) nach Anspruch 1, wobei die Reflexionseinheit (18) an dem Körper (11) durch einen Spindelmotor vorgesehen ist, und der Mikroprozessor (12) konfiguriert ist, um den Spindelmotor gemäß einem in dem Körper (11) vorgesehenen Gyroskop zu steuern, um einen Drehwinkel der Reflexionseinheit (18) zu steuern.

9. Interaktives Anzeigesystem (1), umfassend:
die interaktive Anzeigevorrichtung (10) nach Anspruch 1 und ferner umfassend:
eine drahtlose Übertragungseinheit (16);
wobei das System (1) ferner umfasst:
eine elektronische Vorrichtung (20), die über ein Netzwerk (30) mit der drahtlosen Übertragungseinheit (16) der interaktiven Anzeigevorrichtung (10) verbunden ist, um das Bild zu empfangen und auf einem Bildschirm (22) der elektronischen Vorrichtung (20) anzuzeigen.

10. Interaktives Anzeigesystem (1) nach Anspruch 9, wobei der Körper (11) eine Brille, ein Mobiltelefon oder ein Tablet-Computer ist und die elektronische Vorrichtung (20) ein Mobiltelefon, ein Tablet-Computer oder ein Desktop-Computer ist.

11. Interaktives Anzeigesystem (1) nach Anspruch 9, wobei die optische Projektionseinheit (13) eine lichtemittierende Diode oder ein Laser mit einer Laserlichtquelle und einem Beugungselement ist.

12. Interaktives Anzeigesystem (1) nach Anspruch 11, wobei das Beugungselement ein optisches Gesamtbild oder ein mikrostruktureller optischer Film ist.

13. Interaktives Anzeigesystem (1) nach Anspruch 9, wobei das Muster (44) ein Schachbrettmuster oder ein Muster mit konzentrischen Kreisen ist.

14. Interaktives Anzeigesystem (1) nach Anspruch 9, wobei die Bilderfassungseinheit (14) eine Kamera ist, die einen farbkomplementären Metall-Halbleiter-Bildsensor oder einen farbphotoempfindlichen Koppelelement-Bildsensor beinhaltet.

15. Interaktives Anzeigesystem (1) nach Anspruch 9, wobei das Netzwerk (30) ein drahtloses Netzwerk mit Bluetooth, Wi-Fi, 3G oder 4G ist.

16. Interaktives Anzeigesystem (1) nach Anspruch 9, wobei die Reflexionseinheit (18) an dem Körper (11) durch einen Spindelmotor vorgesehen ist, und der Mikroprozessor (12) konfiguriert ist, um den Spindelmotor gemäß einem in dem Körper (11) vorgesehenen Gyroskop zu steuern, um einen Drehwinkel der Reflexionseinheit (18) zu steuern.

17. Interaktives Anzeigesystem (1) nach Anspruch 9, wobei die elektronische Vorrichtung (20) ein Markierungsmodul (21) umfasst, das konfiguriert ist, um das Bild zu markieren, und wobei das markierte Bild über das Netzwerk (30) an die interaktive Anzeigevorrichtung (10) übertragen und auf der Anzeigeeinheit (15) der interaktiven Anzeigevorrichtung (10) angezeigt werden kann.

## Revendications

1. Dispositif d'affichage interactif (10), comprenant :
un corps (11) dans lequel se trouve un microprocesseur (12) ;
une unité de projection optique (13) prévue sur le corps (11) et commandée par le microprocesseur (12) pour projeter un motif (44) sur une scène (45) ;
une unité de capture d'image (14) prévue sur le corps (11) et commandée par le microprocesseur (12) pour capturer une image contenant le motif (44) dans la scène (45) ;
une unité d'affichage (15) prévue sur le corps (11), configurée pour afficher l'image ;
**caractérisé en ce que** le dispositif d'affichage interactif (10) comprend en outre :
une unité réfléchissante (18) prévue sur le corps (11) et située dans une direction de projection de motif (42) de l'unité de projection optique (13) et une direction de capture d'image (43) de l'unité de capture d'image (14), configurée pour changer la direction de projection de motif (42) et la direction de capture d'image (43).

2. Dispositif d'affichage interactif (10) selon la revendication 1, dans lequel le corps (11) est une lunette, un téléphone mobile ou un ordinateur tablette.

3. Dispositif d'affichage interactif (10) selon la revendication 1, dans lequel l'unité de projection optique (13) est une diode électroluminescente ou un laser ayant une source de lumière laser et un élément de diffraction.

4. Dispositif d'affichage interactif (10) selon la revendication 3, dans lequel l'élément de diffraction est un hologramme optique ou un film optique microstructuré.

5. Dispositif d'affichage interactif (10) selon la revendication 1, dans lequel le motif (44) est un motif en damier ou un motif en cercles concentriques.

6. Dispositif d'affichage interactif (10) selon la revendication 1, dans lequel l'unité de capture d'image (14) est une caméra incluant un capteur d'image à semiconducteur métal complémentaire (CMOS) couleur ou un capteur d'image à élément de couplage photosensible couleur.

7. Dispositif d'affichage interactif (10) selon la revendication 1, comprenant en outre une unité de transmission sans fil (16) configurée pour transmettre l'image à une unité terminale pour affichage, dans lequel l'unité de transmission sans fil (16) utilise une transmission par réseau sans fil Bluetooth, Wi-Fi, 3G ou 4G.

8. Dispositif d'affichage interactif (10) selon la revendication 1, dans lequel l'unité réfléchissante (18) est prévue sur le corps (11) par l'intermédiaire d'un moteur d'axe et le microprocesseur (12) est configuré pour commander le moteur d'axe en fonction d'un gyroscope prévu dans le corps (11) pour commander un angle de rotation de l'unité réfléchissante (18).

9. Système d'affichage interactif (1) comprenant :
le dispositif d'affichage interactif (10) selon la revendication 1 et comprenant en outre :
une unité de transmission sans fil (16) ;
le système (1) comprenant en outre un dispositif électronique (20) connecté à l'unité de transmission sans fil (16) du dispositif d'affichage interactif (10) par l'intermédiaire d'un réseau (30) pour recevoir et afficher l'image sur un écran (22) du dispositif électronique (20).

10. Système d'affichage interactif (1) selon la revendication 9, dans lequel le corps (11) est une lunette, un téléphone mobile ou un ordinateur tablette, et le dispositif électronique (20) est un téléphone mobile, un ordinateur tablette ou un ordinateur de bureau.

11. Système d'affichage interactif (1) selon la revendication 9, dans lequel l'unité de projection optique (13) est une diode électroluminescente ou un laser ayant une source de lumière laser et un élément de diffraction.

12. Système d'affichage interactif (1) selon la revendication 11, dans lequel l'élément de diffraction est un hologramme optique ou un film optique microstructuré.

13. Système d'affichage interactif (1) selon la revendication 9, dans lequel le motif (44) est un motif en damier ou un motif en cercles concentriques.

14. Système d'affichage interactif (1) selon la revendication 9, dans lequel l'unité de capture d'image (14) est une caméra incluant un capteur d'image à semiconducteur métal complémentaire (CMOS) couleur ou un capteur d'image à élément de couplage photosensible couleur.

15. Système d'affichage interactif (1) selon la revendication 9, dans lequel le réseau (30) est un réseau sans fil Bluetooth, Wi-Fi, 3G ou 4G.

16. Système d'affichage interactif (1) selon la revendication 9, dans lequel l'unité réfléchissante (18) est prévue sur le corps (11) par l'intermédiaire d'un moteur d'axe et le microprocesseur (12) est configuré pour commander le moteur d'axe en fonction d'un gyroscope prévu dans le corps (11) pour commander un angle de rotation de l'unité réfléchissante (18).

17. Système d'affichage interactif (1) selon la revendication 9, dans lequel le dispositif électronique (20) comprend un module de marquage (21) configuré pour marquer l'image et l'image marquée peut être transmise au dispositif d'affichage interactif (10) par l'intermédiaire du réseau (30) et affichée sur l'unité d'affichage (15) du dispositif d'affichage interactif (10).
